# EUROPÄISCHE PATENTSCHRIFT

(11) **EP 1 581 271 B1**
(45) Veröffentlichungstag und Bekanntmachung des Hinweises auf die Patenterteilung: **24.05.2006**
(21) Anmeldenummer: 03810842.9
(22) Anmeldetag: 17.12.2003
(51) Int. Cl.: A61L 27/14, A61L 27/50, A61L 31/04, C08L 33/14, C08F 265/04

(54) **PHOTOSENSITIVE POLYMERE NETZWERKE**
PHOTOSENSITIVE POLYMER NETWORKS
RESEAUX POLYMERES PHOTOSENSIBLES

(30) Priorität: 08.01.2003 DE 10300271
(43) Veröffentlichungstag der Anmeldung: 05.10.2005
(73) Patentinhaber: Mnemoscience GmbH, 52531 Uebach-Palenberg (DE)
(72) Erfinder: LENDLEIN, Andreas, 14167 Berlin (DE); JIANG, Hongyan, 52062 Aachen (DE); JÜNGER, Oliver, 55128 Mainz (DE)
(74) Vertreter: Grünecker, Kinkeldey, Stockmair & Schwanhäusser Anwaltssozietät
(86) Internationale Anmeldenummer: PCT/EP2003/014414
(87) Internationale Veröffentlichungsnummer: WO 2004/062706

(56) Entgegenhaltungen:
- WO-A-99/42147
- WO-A-99/42528

## Beschreibung

Die vorliegende Erfindung betrifft photosensitive polymere Netzwerke, photosensitive Komponenten nützlich für die Herstellung der photosensitiven polymeren Netzwerke sowie Programmierungsverfahren.

### Stand der Technik

Polymere Netzwerke sind wichtige Bausteine in vielen Anwendungsbereichen, in denen die klassischen Netzwerke, wie Metalle, Keramik und Holz, aufgrund ihrer beschränkten physikalischen Eigenschaften nicht mehr ausreichend sind. Polymere Netzwerke haben sich daher ein breites Anwendungsgebiet erobert, nicht zuletzt auch dadurch, dass sich durch Variation der monomeren Bausteine der polymeren Netzwerke die Netzwerkeigenschaften variieren lassen.

Eine insbesonders faszinierende Klasse an polymeren Netzwerken, die in den vergangenen Jahren entwickelt wurden, sind die sogenannten Form-Gedächtnis-Polymere (im folgenden auch Shape Memory Polymere, SMP oder SMP-Materialien genannt), d.h. polymere Netzwerke, die neben ihrer aktuellen, sichtbaren Form eine oder sogar mehrere Formen im "Gedächtnis" behalten können, und diese erst durch äußere Reize, wie Temperaturveränderung gezielt einnehmen. Aufgrund der gezielten Formveränderung sind diese Materialien von hohem Interesse in einer Vielzahl von Bereichen, in denen z.B. eine Größenänderung erwünscht ist. Dies trifft z.B. auf medizinische Implantate zu, die möglichst erst am endgültigen Einsatzort ihre vollständige Größe erreichen sollen, so dass die Einführung dieser Implantate nur minimalinvasive chirurgische Eingriffe erfordert. Solche Materialien sind beispielsweise in den internationalen Patentanmeldungen WO-A-99-42528 und WO-A-99-42147 beschrieben.

Die meisten literaturbekannten Form-Gedächtnis-Polymere sind thermisch stimulierbar. In einigen Anwendungsbeispielen ist eine Temperaturänderung jedoch nicht erwünscht, so dass ein anderer Stimulus wie z.B. Licht besser geeignet scheint. Zum Beispiel erlaubt der Einsatz von biokompatiblen SMP in lebenden Organismen Temperaturerhöhungen von nur wenigen Grad Celsius oberhalb Körpertemperatur.

Höhere Temperaturen schädigen das umliegende Gewebe. Meist sind Werkstoffe ohnehin natürlichen Temperaturschwankungen ausgesetzt. Wird hierbei die Übergangstemperatur des SMP überschritten, wird der Form-Gedächtnis-Effekt möglicherweise unerwünscht ausgelöst.

Ein Ansatz zur Überwindung dieser Problematik ist die Verwendung von photosensitiven SMP. Literaturbekannte Beispiele für photosensitive Polymere beziehen sich meist auf Gele, die durch Lichteinwirkung ihren Quellungsgrad ändern (O. Pieroni, F. Ciardelli, Trends Polym. Sci. 3, 282 (1995); Y. Osada, J.-P. Gong, Adv. Mater. 10, 827 (1998); A. Suzuki, T. Tanaka, Nature 346, 345 (1990)). So läßt sich beispielsweise der Sol/Gel Übergang eines photosensitiven Gels durch Lichteinwirkung auslösen (F.M. Andreopoulos, C.R. Deible, M.T. Stauffer, S.G. Weber, W.R. Wagner, E.J. Beckmann, A.J. Russel, J. Am. Chem. Soc. 118, 6235 (1996)). Ein anderes Beispiel ist die durch Licht steuerbare Permeabilität einer Membran aus einem photosensitiven Hydrogel (F.M. Andreopoulos, E.J. Beckmann, A.J. Russel, Biomaterials 19, 1343 (1998)).

Dieser Vorgang ist lediglich eine dreidimensionale, isotrope reversible Volumenänderung, der nicht geeignet ist, um definierte Formänderungen zu realisieren. Gele sind darüber hinaus aufgrund ihrer geringen mechanischen Stabilität ohnehin für viele Anwendungen nicht geeignet.

Die in WO-A-99-42528 und WO-A-99-42147 beschriebenen SMP sind aus Segmenten aufgebaut. Ihre teilkristalline Morphologie verursacht die Streuung von Licht an ihrer Oberfläche und verhindert eine Photoreaktion im Inneren des Materials. Aufgrund dieser Merkmale sind derartige Materialien nicht durch Lichteinwirkung stimulierbar.

### Aufgabe der Erfindung

Es ist daher die Aufgabe der vorliegenden Erfindung polymere Netzwerke anzugeben, die die Nachteile des Stands der Technik überwinden, d.h. insbesondere mit einem nicht mit der Temperatur verbundenen Stimulus schaltbar sind. Im Unterschied zu Hydrogelen soll das Material eine hohe mechanische Festigkeit aufweisen. Die polymeren Netzwerke sollten darüber hinaus die Möglichkeit eröffnen, dass durch einfache Variation der Zusammensetzung eine Eigenschaftensteuerung möglich wird, wodurch gezielt Materialien mit einem erwünschten Werkstoffprofil erhalten werden können.

### Kurze Beschreibung der Erfindung

Die vorliegende Erfindung löst diese Aufgabe durch das photosensitive polymere Netzwerk nach Anspruch 1. Bevorzugte Ausführungsformen sind in den Unteransprüchen angegeben. Dieses photosensitive polymere Netzwerk ist insbesondere kein Hydrogel.

Darüber hinaus stellt die vorliegende Erfindung photosensitive Komponenten zur Verfügung, die zur Herstellung der polymeren amorphen Netzwerke geeignet sind, beispielsweise in den angegebenen Verfahren.

Schließlich stellt die vorliegende Erfindung ein Verfahren zur Programmierung der photosensitiven SMP zur Verfügung. Bevorzugte Ausführungsformen sind wiederum in den Unteransprüchen angegeben.

Weitere Aspekte der Erfindung sind in den Ansprüchen sowie in der folgenden Beschreibung definiert.

### Kurze Beschreibung der Figuren

Figur 1 zeigt das Funktionsprinzip eines photosensitiven Netzwerks auf makroskopischer und molekularer Ebene.
Figur 2 veranschaulicht die Photoreaktion der Zimtsäure und eines Cinnamylacylats
Figur 3 zeigt das mechanische Verhalten der photosensitiven Netzwerke im zyklischen photomechanischen Experiment.
Figur 4 verdeutlicht die Abhängigkeit der Form-Gedächtnis-Eigenschaften vom Gehalt der photoreaktiven Komponente

### Detaillierte Beschreibung der Erfindung

Im folgenden wird die vorliegende Erfindung detailliert beschrieben.

Das photosensitive polymere Netzwerk im Sinne der Erfindung umfasst ein kovalent vernetztes Polymer (amorphes Netzwerk), das mit photoreaktiven Gruppen ausgerüstet ist (kovalent in das amorphe Netzwerk eingebunden oder physikalisch mit diesem vermischt), welche dem Material lichtinduzierbare Form-Gedächtnis-Eigenschaften verleihen. Das Polymergerüst absorbiert nicht die für die Photoreaktion erforderliche Wellenlänge. Das Netzwerk ist darüber hinaus im wesentlichen amorph, homogen und transparent.

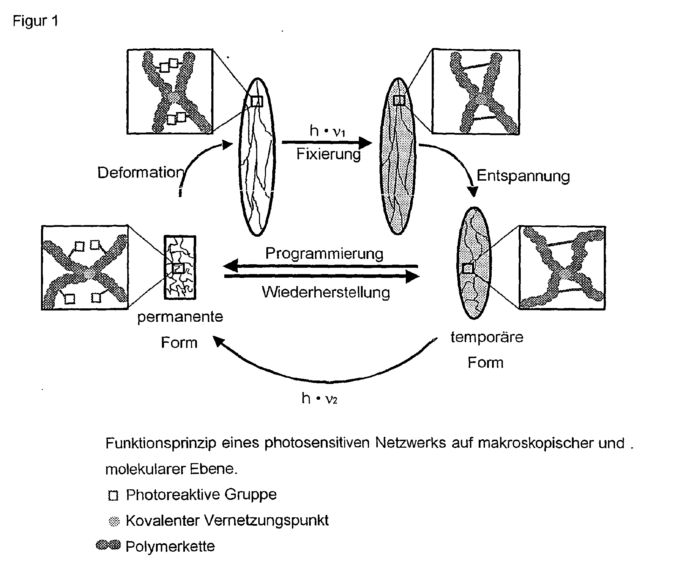

Figur 1 zeigt das Funktionsprinzip eines photosensitiven Netzwerks auf makroskopischer und molekularer Ebene. Entlang der "Hauptketten" des Netzwerks befinden sich Substituenten, die am Ende mit einer photoreaktiven Gruppe ausgestattet sind. Bei UV-Bestrahlung sind diese Gruppen fähig, kovalente Bindungen miteinander einzugehen. Deformiert man das Material und bestrahlt es mit Licht einer geeigneten Wellenlänge λ₁, wird das ursprüngliche Netzwerk zusätzlich quervernetzt. Aufgrund der Vernetzung erreicht man eine temporäre Fixierung des Materials im deformierten Zustand (Programmierung). Da die Photovemetzung reversibel ist, lässt sich durch erneutes Bestrahlen mit Licht einer anderen Wellenlänge λ₂ die Vernetzung wieder lösen und somit die ursprüngliche Form des Materials wieder abrufen (Wiederherstellung). Ein solcher photomechanischer Zyklus lässt sich beliebig oft wiederholen.

Damit die erfindungsgemäßen photosensitiven polymeren Netzwerke die gewünschten Eigenschaften zeigen, d.h. um die gestellte Aufgabe zu erfüllen, muss das Netzwerk im wesentlichen transparent für die zur Formveränderung gedachten Strahlung sein. Üblicherweise liegt diese Strahlung im UV-Bereich, da so insbesondere auch eine Auslösung der Formveränderung durch sichtbares Licht, das in allen Lebensbereichen nur schwer vollständig auszuschließen ist, vermieden werden kann. Darüber hinaus ist der ebenfalls in den meisten Lichtquellen enthaltene Anteil an UV-Strahlung nicht ausreichend, um eine Formveränderung im erfindungsgemäßen Material auszulösen. Bevorzugt ist also das Material der vorliegenden Erfindung transparent gegenüber UV-Strahlung, insbesondere im Bereich von 200 bis 400 nm, stärker bevorzugt im Bereich von 250 bis 350 nm.

### Die Komponenten der Netzwerke

### 1. Matrix

Die Basis der Netzwerke ist durch eine Matrix geformt, die, wie vorstehend ausgeführt, transparent im Hinblick auf die zur Auslösung der Formveränderung gedachten Strahlung ist, d.h. bevorzugt eine UV-transparente Matrix. Weiterhin sollte diese Matrix eine gewissen Grad an Elastizität und Flexibilität aufweisen (elastomere Eigenschaften). Darüber hinaus ist es erforderlich, dass die Matrix amorph ist. Schließlich ist es wichtig, dass die Matrix vernetzt ist, um ein gewisses Maß an mechanischer Stabilität sicherzustellen, ebenso wie die erwünschten erfindungsgemäßen Form-Gedächtnis-Eigenschaften. Prinzipiell sind in diesem Sinne alle polymerisierbaren Verbindungen erfindungsgemäß einsetzbar, die eine solche Matrix ergeben, wobei bevorzugt diese Verbindungen in Masse polymerisierbar sein sollten.

Erfindungsgemäß bevorzugt ist für die Basis des Netzwerks der vorliegenden Erfindung eine Matrix auf Grundlage von niedermolekularen Acrylaten und Methacrylaten, die sich radikalisch polymerisieren lassen, insbesondere C1-C6-(meth)Acrylate und Hydroxyderivate davon, wobei Hydroxyethylacrylat, Hydroxypropylmethacrylat, Hydroxypropylacrylat, Poly(ethylenglycol)methacrylat und n-Butylacrylat bevorzugt sind; vorzugsweise werden n-Butylacrylat und Hydroxyethylmethacrylat verwendet.

n-Butylacrylat, das als Matrixkomponente bevorzugt ist hat den Vorteil, dass sein Homopolymer eine tiefe Glastemperatur von -55 °C aufweist, sodass man aufgrund dieser Komponente in den Netzwerken elastische Eigenschaften erwarten kann. Ein Comonomer, bevorzugt Hydroxyethylmethacrylat, dient ggf. zur Steuerung der thermischen und mechanischen Eigenschaften. Diese beiden Verbindungen können in beliebigen Verhältnissen polymersisiert werden, wobei, wenn Hydroxyethylmethacrylat (HEMA) vorliegt, n-Butylacrylat den Hauptanteil ausmacht. Bevorzugte Molverhältnisse von n-Butylacrylat zu HEMA liegen im Bereich von 10:0,1 bis 10:5, bevorzugt 10:1 bis 10:3 und insbesondere etwa 10:2.

### 2. Vernetzer

Neben dem Material für die Matrix enthält das polymere Netzwerk der vorliegenden Erfindung auch eine Komponente, die für die Vernetzung der Matrix verantwortlich ist. Die chemische Natur dieser Komponente hängt natürlich von der Natur der Matrixmaterialien ab. Auch hier sind wieder eine Vielzahl von Verbindungen einsetzbar, abgestimmt mit den Matrixmaterialien.

Für die bevorzugten Netzwerke auf der Basis der oben als bevorzugt beschriebenen Acrylatmaterialien sind geeignete Vernetzer bifunktionelle Acrylatverbindungen, die mit den Ausgangsmaterialien für die Matrix geeignet reaktiv sind, so dass sie gemeinsam umgesetzt werden können. Derartige Vernetzer umfassen kurze, bifunktionelle Vernetzer, wie Ethylendiacrylat, niedermolekulare bi- oder polyfunktionelle Vernetzer, oligomere, lineare Diacrylatvernetzer, wie Poly(oxyethylen)diacrylaten oder Poly(oxypropylen)diacrylaten, und verzweigte Oligomere oder Polymere mit Acrylatendgruppen.

Als Vernetzer dient bevorzugt ein Dimethacrylat, insbesondere Poly(propylenglycol)-dimethacrylat mit einem Molgewicht von 300 bis 1000 g/mol, bevorzugt von etwa 560 g/mol. Der Vernetzer wird in relativ geringen Konzentrationen von etwa 0,3 bis 3 mol% eingesetzt, bezogen auf die Gesamtmenge an Materialien, die zum Netzwerk polymerisiert werden sollen, damit elastische Netzwerke entstehen. Größere Vernetzeranteile führen zu weniger elastischen bis hin zu spröden Materialien.

Erfindungsgemäß erfolgt die Einbringung der Vernetzer in das Netzwerk durch einfaches mischen der Vernetzer mit den Ausgangsmaterialein für die Matrix und anschließendes Polymerisieren, bevorzug in Masse, mit geeigneten Initiatoren.

### 3. Photoreaktive Komponente

Als weitere Komponente umfasst das erfindungsgemäße Netzwerk eine photoreaktive Komponente (Gruppe), die für die Auslösung der gezielt steuerbaren Formveränderung mitverantwortlich ist. Diese photoreaktive Gruppe ist eine Einheit, die durch Anregung mit einer geeigneten Lichtstrahlung, bevorzugt UV-Strahlung zu einer reversiblen Reaktion fähig ist (mit einer zweiten photoreaktiven Gruppe), die zur Erzeugung oder Lösung von kovalenten Bindungen führt. Bevorzugte photoreaktive Gruppen sind solche, die zu einer reversiblen Photodimerisierung fähig sind.

Die photoreaktive Komponente kann bei geeigneter Funktionalisierung entweder direkt mit den genannten Monomeren radikalisch copolymerisiert werden oder den interpenetrierenden Teil eines interpenetrierenden Netzwerks IPN bilden.

Geeignete photoreaktive Komponenten sind solche, die die oben genannten Eigenschaften aufweisen und darüber hinaus entweder mit in ein Netzwerk einpolymerisiert werden können (z.b. in ein Acrylat-haltiges Netzwerk durch Einführung der photoreaktiven Gruppe in ein Acrylatmonomer oder -oligomer) oder durch Quellverfahren oder Ähnliches in ein bereits bestehendes Netzwerk eingeführt werden können, z.B. in der Form von geeignet funktionalisierten Polymeren oder Oligomeren.

Als photoreaktive Komponenten in den erfindungsgemäßen photosensitiven Netzwerken dienen bevorzugt verschiedene Zimtsäureester (Cinnamate, CA) und Cinnamylacylsäureester (Cinnamylacylate, CAA).

Es ist bekannt, dass Zimtsäure und ihre Derivate unter UV-Licht von etwa 300 nm unter Ausbildung eines Cydobutans dimerisieren. Die Dimere lassen sich wieder spalten, wenn mit UV-Licht einer kleineren Wellenlänge von etwa 240 nm bestrahlt wird. Die Absorptionsmaxima lassen sich durch Substituenten am Phenylring verschieben, verbleiben aber stets im UV-Bereich. Weitere Derivate, die sich photodimerisieren lassen, sind 1,3-Diphenyl-2-propen-1-on (Chalkon), Cinnamylacylsäure, 4-Methylcoumarin, verschiedene ortho-substituierte Zimtsäuren, Cinammyloxysilane (Silylether des Zimtalkohois).

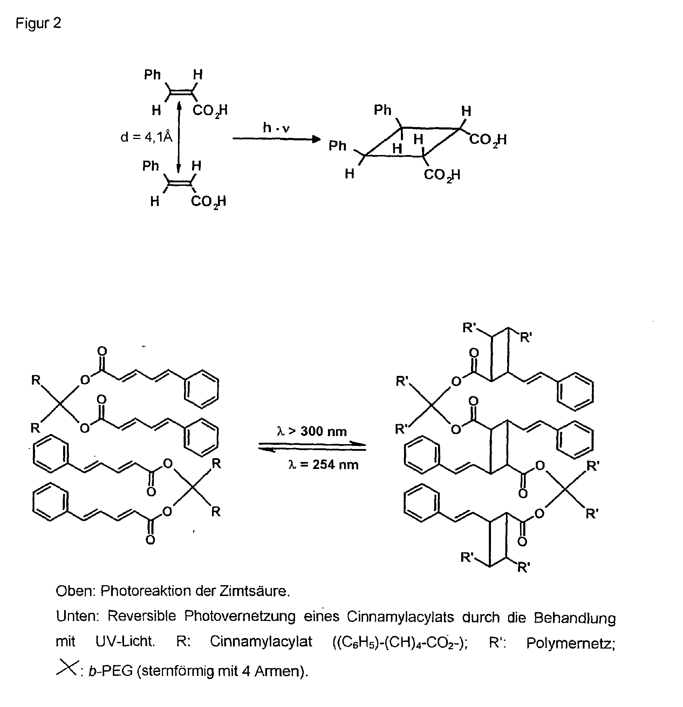

Bei der Photodimerisierung von Zimtsäure und ähnlichen Derivaten handelt es sich um eine [2+2] Cycloaddition der Doppelbindungen zu einem Cyclobutanderivat. Sowohl die E- als auch Z-Isomere sind in der Lage, diese Reaktion einzugehen. Unter Bestrahlung läuft die E/Z-Isomerisierung in Konkurrenz zur Cycloaddition ab. Im kristallinen Zustand ist die E/Z-Isomerisierung jedoch gehindert. Aufgrund der verschiedenen Anordnungsmöglichkeiten der Isomere zueinander sind theoretisch 11 verschiedene stereoisomere Produkte (Truxillsäuren, Truxinsäuren) möglich. Der für die Reaktion erforderliche Abstand der Doppelbindungen zweier Zimtsäuregruppen beträgt etwa 4 Å. Figur 2 veranschaulicht die Photoreaktion der Zimtsäure und eines Cinnamylacylats.

Die Einführung der photoreaktiven Komponente in das erfindungsgemäße Netzwerk erfolgt, wie vorstehend beschrieben, auf zwei unterschiedlichen Wegen. Einerseits kann die photoreaktive Gruppe (Komponente) mit in die Matrix des Netzwerks einpolymerisiert werden, so dass das Netzwerk als solches photoreaktiv ist. Dies vereinfacht in gewisser Weise das Herstellungsverfahren, da nach einer einzigen Polymerisation das polymere photosensitive Netzwerk erhalten werden kann. Ggf. erforderliche weitere Reaktionsschritte betreffen dann nur noch entweder Reinigungsschritte oder Schritte zur Einführung weiterer optionaler Komponenten. Gleichzeitig lassen sich so die Eigenschaften des erfindungsgemäßen Netzwerks einfach steuern, da im wesentlichen die Polymerisationsmischung bereits diese bestimmt. Die zweite Alternative besteht darin, dass nicht das Netzwerk als solches mit der photoreaktiven Gruppe ausgestattet wird sondern dass diese durch physikalische Verfahren mit der Netzwerkmatrix gemischt wird. Ein typisches Beispiel dafür ist die Herstellung eines IPN aus vernetzter Polymermatrix (die wie vorstehend beschrieben sein kann) mit einem geeignet funktionalisierten zweiten Polymer oder Oligomer, das die photoreaktive Gruppe trägt und das Netzwerk durchdringen kann. Ein Vorteil dieser Variante besteht darin, dass die Herstellung der polymeren Netzwerkmatrix nicht so strengen Einschränkungen unterliegt, da die empfindliche und bei bestimmten Polymerisierungsverfahren störende photoreaktive Gruppe bei der Herstellung der Netzwerkmatrix nicht vorliegt. So kann z.B. in diesem Fall die Netzwerkmatrix durch UV-Initiierung polymersiert werden, was bei der ersten Alternative nicht möglich ist, da dann die photoreaktiven Guppen der photoreaktiven Komponente störend in die Polymerisation eingreifen können.

Zum Nachweis der Photoreaktion (Cycloaddition) können verschiedene spektroskopische Methoden zu Rate gezogen werden. UV-spektroskopisch beobachtet man eine Abnahme des Absorptionsmaximums bei 275 nm infolge Aufhebung der Konjugation der π-Elektronen des Benzolrings mit der Alken-Carbonyl-Gruppe.

### 3.1 Einpolymerisierung der photoreaktiven Komponente

Eine Möglichkeit zur Einführung der photoreaktiven Komponente in ein Netzwerk ist es, die photoreaktive Gruppe an Ausgangsmaterialien der Netzwerkmatrix zu koppeln. Bei den Bevorzugten Netzwerken auf Basis von Acrylaten besteht dabei z.B. die Möglichkeit, die entsprechenden Zimtsäure- oder Cinnamylacylsäurechloride mit Hydroxyalkylacrylaten bzw. -methacrylaten zu verestem. Dadurch erhält man photoreaktive Ester, die einfach mit anderen Monomeren radikalisch copolymerisiert werden können. Geeignete Hydroxyacrylate und -methacrylate für die Veresterung mit Zimtsäure (CA) oder Cinnamylacylsäure (CAA) sind Hydroxyethylmethacrylat (HEMA), Hydroxyethylacrylat (HEA), Hydroxypropylmethacrylat (HPMA), Hydroxypropylacrylat (HPA), Poly(ethylenglycol)methacrylat (PEGMA). Die Veresterung erfolgt unter Bedingungen, die dem Fachmann aus dem Stand der Technik bekannt sind (Methode nach Schotten-Baumann. Das Hydroxyalkyacrylat oder -methacrylat wird in Diethylether gelöst und zuerst mit z.B. Zimtsäurechlorid, dann mit Triethylamin versetzt.)

Die radikalische Polymerisation der oben genannten Komponenten zum Netzwerk erfolgt vorzugsweise in Substanz unter Zusatz eines thermolabilen Initiators. Geeignete Initiatoren sind Peroxide wie Benzoylperoxid, Di-tert.-butylperoxid, und Azoverbindungen wie Azobisisobutyronitril (AiBN). Bevorzugt wird AiBN in Konzentrationen von 0,1 bis 1 gew% eingesetzt.

Die Menge an photoreaktiver Komponente beträgt üblicherweise von 1 bis 30 Mol-%, bezogen auf die Gesamtmischung aus 1 . bis 3., bevorzugt 2 bis 20 Mol-%, stärker bevorzugt 4 bis 12 Mol%.

Die Einpolymerisierung ergibt eine statistische Verteilung der photoreaktiven Komponente im polymeren Netzwerk, wie durch spektroskopische Untersuchungen gezeigt werden konnte. Dies bewirkt eine Sicherung der Form-Gedächtnis-Eigenschaften, da lediglich bei einer gleichmäßigen Verteilung der photoreaktiven Komponente im Gesamtnetzwerk einheitliche, reproduzierbare und verlässliche Form-Gedächtnis-Eigenschaften erwartet werden können.

### 3.2 Nachträgliche Beladung (physikalische Vermischung)

Eine andere Möglichkeit ein Netzwerk mit photoreaktiven Gruppen auszustatten, ist die nachträgliche physikalische Beladung nicht funktionalisierter Netzwerke. Die Beladung eines Netzwerkes erfolgt, indem es in einer Lösung der photoreaktiven Komponente aufgequollen und dann getrocknet wird. Die photoreaktive Komponente durchdringt somit das gesamte Netzwerk. Wird das beladene Netzwerk anschließend mit UV-Licht bestrahlt, dimerisieren die photoreaktiven Gruppen unter Ausbildung eines reversiblen Netzwerks im permanenten Netzwerk. Es entsteht ein interpenetrierendes Netzwerk (IPN).

Das nicht funktionalisierte Netzwerk dieser Ausführungsform entspricht bevorzugt dem amorphen Netzwerk, das oben beschrieben wurde und eine Matrixkomponente und eine Vernetzerkomponente umfasst. Die oben in diesem Zusammenhang angeführten bevorzugten Ausführungsformen gelten auch hier als bevorzugt.

Damit überhaupt ein reversibles Netzwerk entstehen kann, ist es erforderlich, dass die photoreaktive Komponente mindestens drei photovernetzbare Gruppen pro Molekül enthält. Zur Beladung der permanenten Netzwerke eignen sich somit insbesondere sternförmige, verzweigte Polymere oder Oligomere oder kamm- oder stabartige Pfropfpolymere oder -oligomere. Bevorzugt wird ein sternförmiges Makromonomer mit einer photoreaktiven Gruppe an jedem Kettenende (Arm) verwendet. Die Arme bestehen dabei bevorzugt aus Alkylenglycoleinheiten.

Das Makromonomer lässt sich aus sternförmigen Molekülen mit endständigen OH-Gruppen herstellen, die mit einem der oben genannten photoreaktiven Säurechloride verestert werden. Bevorzugt wird ein vierarmiges Poly(ethylenglycol) mit einem Molgewicht von 400 bis 1000 g/mol, bevorzugt etwa 560 g/mol verwendet, welches kommerziell erhältlich ist. Das Molgewicht und die Zahl der Arme sind dabei aber nicht entscheidend. Es sind jedoch mindestens drei Arme erforderlich. Die Veresterung erfolgt unter literaturbekannten Bedingungen.

Die Beladung des Netzwerks mit der photoreaktiven Komponente erfolgt durch Quellen des Netzwerks in einer Lösung der photoreaktiven Komponente. Bei den bevorzugten Netzwerken auf der Basis von Acrylaten, beladen mit der bevorzugten vierarmigen sternförmigen photoreaktiven Komponente, beträgt die Beladung vorzugsweise von 5 bis 45 Gew.-%, bezogen auf die Gesamtmischung, stärker bevorzugt 15 bis 35 Gew.-% und insbesondere 25 bis 35 Gew.-%, am meisten bevorzugt etwa 30 Gew.-%.

Auch bei den erfindungsgemäß bevorzugten IPN liegt die photoreaktive Komponente im wesentlichen in einer einheitlichen Verteilung im Netzwerk vor, was, wie oben beschrieben, die Form-Gedächtnis-Eigenschaften sicherstellt.

### Die photosensitiven Netzwerke

### Einfache Netzwerke

Durch radikalische Copolymerisation eines Zimtsäureesters, wie oben angegeben, mit Acrylaten oder Methacrylaten, wie oben aufgeführt, gelingt die Darstellung photoreaktiver Netzwerke, wie am Beispiel zweier Netzwerk-Serien verdeutlicht werden soll. In der ersten Serie wurde einer der Zimtsäureester copolymerisiert mit zwei Komponenten (n-Butylacrylat und Poly(propylenglycol)dimethycrylat), in der zweiten Serie mit drei Komponenten (zusätzlich Hydroxyethylmethacrylat HEMA). Die Konzentration des Zimtsäureestes wurde innerhalb einer Reihe variiert. Der Gehalt an photoreaktiver Komponente in den Mischungen betrug zwischen 0,075 und 1,27 mmol/g.

Die Werte für den Gelgehalt der erhaltenen Netzwerke, d.h. der Anteil an nicht extrahierbaren Bestandteilen, liegen oft oberhalb von 90 %, meist sogar oberhalb von 95 %, was hohen Umsätzen entspricht. Man kann daher näherungsweise annehmen, dass eine Monomermischung und das korrespondierende Netzwerk dieselbe Zusammensetzung aufweisen.

### IPN

Zur physikalischen Beladung mit photosensitiven Komponenten (Makromonomer) geeignete Netzwerke bestehen vorzugsweise aus n-Butylacrylat und Poly(propylenglycol)dimethacrylat. Die Netzwerke werden in einer Lösung des Makromonomers in THF aufgequollen und wieder getrocknet. Der Beladungsgrad kann über die Konzentration der Lösung gesteuert werden. Nach dem Trocknen der getränkten Proben lässt sich eine Gewichtszunahme von beispielsweise 30 % feststellen, wenn die Lösung 10 gew% Makromonomer enthielt. Das entspricht einem Gehalt an photoreaktiven Gruppen im Netzwerk von 0,32 mmol/g (0,32 mmol/g x 85% Endgruppenfunktionalisierung = 0,27 mmol/g).

Diese photosensitiven Netzwerke der vorliegenden Erfindung zeichnen sich durch die folgenden Eigenschaften aus.

Alle Netzwerke sind transparent, was für eine homogene, amorphe Morphologie spricht. Eine Ausnahme stellen die Netzwerke 10A-C dar; die leicht opak sind.

Die Netzwerke zeichnen sich durch eine tiefe Glastemperatur aus. Für die Netzwerke der Reihe ohne HEMA liegt sie zwischen - 46,1 und -10,9 °C (DSC), mit HEMA zwischen -11,9 und 16,1 °C. Tendenziell steigt die Glastemperatur mit zunehmendem Gehalt an photoreaktiver Komponente.

Oberhalb ihrer Glastemperatur sind die Netzwerke elastisch. Bei Raumtemperatur beträgt die Reißspannung der meisten Netzwerke ohne HEMA 20 - 45 %, die der Netzwerke mit HEMA bis zu 60 %. Der E-Modul steigt tendenziell mit zunehmendem Anteil an photoreaktivem Comonomer im Netzwerk auf Werte bis 4,2 MPa (Netzwerke ohne HEMA) bzw. bis 120 MPa (mit HEMA), d.h. die Elastizität nimmt ab. Die interpenetrierenden Netzwerke lassen sich um mehr als 100 % dehnen, ohne zu reißen.

Durch die Photoreaktion verändern sich die mechanischen Eigenschaften des Materials. Die UV-Bestrahlung mit λ₁ bewirkt eine kovalente Vernetzung der photoreaktiven Gruppen und kann eine Erhöhung des E-Moduls um 18 % bewirken (Beispiel IPN). Bei Bestrahlung mit UV-Licht einer anderen charakteristischen Wellenlänge λ₂ wird die Vernetzung aufgehoben und der E-Modul nimmt wieder ab.

Die hohe Elastizität der Netzwerke vor der Bestrahlung ermöglicht die leichte Umformung des Materials für die Programmierung einer temporären Form. Insgesamt sind die amorphen Netzwerke der vorliegenden Erfindung gute SMP-Materialien, mit hohen Rückstellwerten, d.h. die ursprüngliche Form wird auch beim Durchlaufen mehrerer Zyklen an Formänderungen zu einem hohen Prozentsatz, üblicherweise oberhalb von 90%, erneut erhalten. Dabei tritt auch kein nachteiliger Verlust an mechanischen Eigenschaftswerten auf.

Die Form-Gedächtnis-Eigenschaften der Materialien der vorliegenden Erfindung werden nachfolgend kurz definiert.

Form-Gedächtnis-Polymere im Sinne der vorliegenden Erfindung sind Materialien, die durch ihre chemisch-physikalische Struktur in der Lage sind, gezielte Formänderungen durchzuführen. Die Materialien besitzen neben ihrer eigentlichen permanenten Form eine weitere Form, die dem Material temporär aufgeprägt werden kann. Solche Materialien sind durch zwei Merkmale charakterisiert. Sie umfassen sogenannte photoreaktive Gruppen, die einen durch Licht stimulierten Übergang auslösen können. Darüber hinaus umfassen diese Materialien kovalente Vernetzungspunkte, die für die sogenannte permanente Form verantwortlich sind. Diese permanente Form wird durch die dreidimensionale Struktur eines Netzwerks gekennzeichnet. Die im erfindungsgemäßen Netzwerk vorliegenden Vemetzungspunkte sind kovalenter Natur und werden in den bevorzugten Ausführungsformen der vorliegenden Erfindung erhalten durch die Polymerisation der Acrylat- bzw Methacrylatgruppen. Die photoreaktiven Gruppen, die den durch Licht induzierten Übergang (Formveränderung) auslösen, sind in der vorliegenden Erfindung, bezogen auf die bevorzugten Ausführungsformen, die Cinnamatgruppen bzw. Cinnamylacylgruppen.

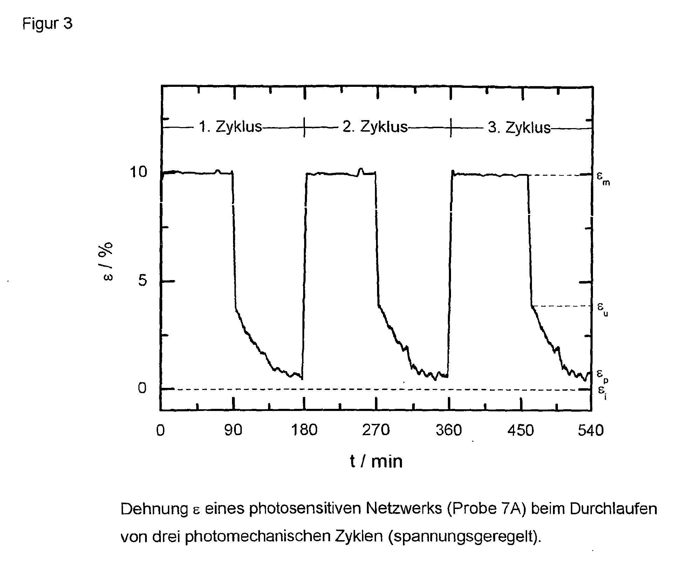

Ein photomechanischer Zyklus besteht aus den Schritten: Dehnung der Probe, Bestrahlung mit λ₁ (Fixierung, Programmierung), Entspannung der Probe, Bestrahlung mit λ₂ (Wiederherstellung). Durch geeignete Zug-Dehnungs-Experimente kann der Form-Gedächtnis-Effekt gezeigt werden. Als Beispiel für solche Zug-Dehnungs-Messungen zeigt Figur 3 das mechanische Verhalten eines photosensitiven Netzwerks beim Durchlaufen von drei photomechanischen Zyklen.

In Figur 3 wurde eine SMP Folie um 10% gedehnt (von εₗ auf εₘ) und 90 Minuten mit λ₁ > 250 nm bestrahlt (je 45 min pro Seite). Die Genauigkeit, mit der die temporäre Form fixiert werden kann, bezeichnet man als Formfixierung R_{f}. Die Klemmen wurden dann auf die Ausgangslänge zurückgebracht (εᵤ) und die (jetzt gebogene) Folie im spannungsfreien Zustand erneut 90 Minuten mit λ₂ < 250 nm bestrahlt. Dabei zieht sich die Folie wieder zusammen (Form-Gedächtnis-Effekt), wobei im ersten Zyklus nicht exakt die ursprüngliche Länge erreicht wird, sondern eine geringe Restdehnung im Material verbleibt (εₚ) (Equilibrierung in den ersten Zyklen). Die Genauigkeit, mit der die ursprüngliche Form wieder erhalten wird, wird als Rückstellverhältnis Rᵣ bezeichnet.
R_{f} und Rᵣ berechnen sich nach: (a) R_{f} = εᵤ / εₘ x 100
und (b) Rᵣ (N) = (εₘ - εₚ (N))/εₘ- εₚ (N-1) x 100
mit N = Nummer des Zyklus.

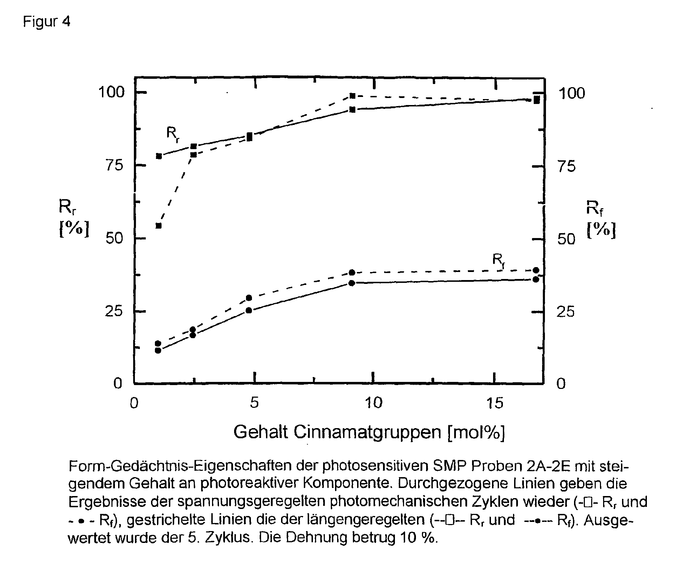

Die Bestrahlung der gedehnten Probe kann entweder längengeregelt (konstante Probenlänge) oder spannungsgeregelt (konstante Spannung) durchgeführt werden. Hält man die Dehnung während der Bestrahlung konstant, nimmt die Spannung zu. Bei konstant gehaltener Spannung ist im Allgemeinen ein Zusammenziehen der Proben festzustellen. Figur 4 verdeutlicht, dass die Verfahrensweise nur einen geringen Einfluß auf die Form-Gedächtnis-Eigenschaften hat. Die Form-Gedächtnis-Eigenschaften sind abhängig von der Konzentration der photoreaktiven Gruppen im Netzwerk, wie Figur 4 weiterhin aufzeigt. Rᵣ und R_{f} (ausgewertet wurde der 5. Zyklus) erreichen bei einer Konzentration von ca. 18 % einen Grenzwert.

Die erfindungsgemäßen photosensitiven polymeren Netzwerke zeichnen sich dadurch aus, dass zum Ersten mal funktionsfähige Form-Gedächtnis-Materialien geschaffen wurden, die mit einem Stimulus geschaltet werden können, der von der Temperatur verschieden ist. Damit eröffnet die vorliegende Erfindung einneues Gebiet der Form-Gedächtnis-Materialien und einen neuen Weg für den Einsatz solcher Materialien in Bereichen in denen Temperatur gesteuerte Form-Gedächtnis-Materialien nicht eingesetzt werden können. Die bevorzugten Netzwerke der vorliegenden Erfindung können darüber hinaus mit UV-Licht in einem eng umgrenzten Wellenlängenbereich geschaltet werden, ein Bereich der für viele Anwendungen problemlos ist, da zum einen entsprechende Strahlungsquellen bereits vorhanden sind und weiterhin dieser Wellenlängenbereich für andere Materialien ohne Nachteil ist.

Die amorphen Netzwerke der vorliegenden Erfindung können, neben den oben diskutierten wesentlichen Komponenten weitere Stoffe enthalten, solange die Funktion der Netzwerke nicht beeinträchtigt wird. Solche zusätzlichen Materialien können beispielsweise Färbmittel, Füllstoffe oder zusätzliche polymere Materialien sein, die für verschiedene Zwecke eingesetzt werden können. Insbesondere für medizinische Zwecke einzusetzende amorphe Netzwerke der vorliegenden Erfindung können medizinische Wirkstoffe und Diagnostika, wie Kontrastmittel umfassen. Diese können durch bekannte Verfahren in das Netzwerk eingebracht werden.

Die folgenden Anwendungsbeispiele erläutern die Erfindung.

### Herstellung der sternförmigen photosensitiven Makromonomere

Sternförmiges Poly(ethylenglycol) mit 4 Armen (Molgewicht 2000 g/mol) wird in trockenem THF und Triethylamin gelöst. Dazu wird langsam in trockenem THF gelöstes Cinnamyliden acetylchlorid getropft. Das Reaktionsgemisch wird für 12 Stunden bei Raumtemperatur, dann für 3 Tage bei 50 °C gerührt. Ausgefallene Salze werden abfiltriert, das Filtrat aufkonzentriert und das erhaltene Produkt mit Diethylether gewaschen. H-NMR Messungen ergeben einen Umsatz von 85 %. UV-spektroskopisch weist das Makromonomer vor der Photoreaktion ein Absorptionsmaximum bei 310 nm, nach der Photoreaktion bei 254 nm auf.

### Herstellung der Netzwerke

10 mmol n-Butylacrylat (BA), ein Zimtsäureester (0,1 - 3 mmol) und ggf. 2 mmol Hydroxyethylmethacrylat (HEMA) werden in einem Glaskolben vermischt. Zur Mischung werden 1 mol% AiBN und 0,3 mol% Poly(propylenglycol)dimethacrylat (Mn = 560) hinzugefügt. Die Mischung wird mit einer Spritze in eine Form aus zwei silylierten Objektträgern, zwischen denen sich ein Teflondichtring einer Dicke von 0,5 mm befindet, gefüllt. Die Polymerisation der Mischung erfolgt 18 Stunden bei 80 °C.

Die Form in der die Vernetzung erfolgt entspricht der permanenten Form. Die Mischung lässt sich auch in beliebigen anderen Formen vernetzen.

Nach der Polymerisation wird das Netzwerk aus der Form gelöst und mit 150 mL Hexan-Fraktion bedeckt. Dann wird nach und nach Chloroform zugegeben. Dieses Lösungsmittelgemisch wird innerhalb von 24 Stunden mehrmals ausgetauscht, um niedermolekulare und unvernetzte Bestandteile herauszulösen. Abschließend wird das Netzwerk mit Hexan-Fraktion gereinigt und im Vakuum bei 30 °C über Nacht getrocknet. Das Gewicht der extrahierten Probe relativ zum vorherigen Gewicht entspricht dem Gelgehalt. Die beiden nachfolgenden Tabellen zeigen die Mengen der verwendeten Monomere sowie die Quellung der Netzwerke in Chloroform und deren Gelgehalt.

| Nr. | Monomergehalt der Mischung (mmol) | | | | | | Q (%) | G (%) |
|---|---|---|---|---|---|---|---|---|
| | BA | HEMA-CA | HEA-CA | HPMA-CA | HPA-CA | PEGMA-CA | | |
| 1A | 10 | 0,25 | - | - | - | - | 720 | 97,2 |
| 1 B | 10 | 0,5 | - | - | - | - | 550 | 94,9 |
| 1C | 10 | 1 | - | - | - | - | 400 | 91,6 |
| 2A | 10 | - | 0,1 | - | - | - | 620 | 89,0 |
| 2B | 10 | - | 0,25 | - | - | - | 900 | 96,2 |
| 2C | 10 | - | 0,5 | - | - | - | 680 | 95,7 |
| 2D | 10 | - | 1 | - | - | - | 1320 | 96,5 |
| 2E | 10 | - | 2 | - | - | - | 1320 | 96,5 |
| 3A | 10 | - | - | 0,25 | - | - | 950 | 98,7 |
| 3B | 10 | - | - | 0,5 | - | - | 650 | 93,4 |
| 3C | 10 | - | - | 1 | - | - | 450 | 98,4 |
| 4A | 10 | - | - | - | 0,25 | - | 830 | 95,9 |
| 4B | 10 | - | - | - | 0,5 | - | 700 | 98,1 |
| 4C | 10 | - | - | - | 1 | - | 550 | 94,3 |
| 5A | 10 | - | - | - | - | 0,25 | 600 | 98,2 |
| 5B | 10 | - | - | - | - | 0,5 | 550 | 97,3 |
| 5C | 10 | - | - | - | - | 1 | 530 | 92,4 |

In einer weiteren Serie wird den binären Polymersystemen zusätzlich ein Anteil von 2 mmol Hydroxyethylmethacrylat (HEMA) zugefügt, da durch dieses Comonomer eine weitere Möglichkeit zur Kontrolle der mechanischen Eigenschaften der Polymernetzwerke zu erwarten ist.

| Nr. | Monomergehalt der Mischung (mmol) | | | | | | | Q (%) | G (%) |
|---|---|---|---|---|---|---|---|---|---|
| | BA | HEMA | HEMA-CA | HEA-CA | HPMA-CA | HPA-CA | PEGMA-CA | | |
| 6A | 10 | 2 | 1 | - | - | - | - | 370 | 95,5 |
| 6B | 10 | 2 | 2 | - | - | - | - | 350 | 99,2 |
| 6C | 10 | 2 | 3 | - | - | - | - | 420 | 96,8 |
| 7A | 10 | 2 | - | 1 | - | - | - | 390 | 98,5 |
| 7B | 10 | 2 | - | 2 | - | - | - | 300 | 92,8 |
| 7C | 10 | 2 | - | 3 | - | - | - | 250 | 96,4 |
| 8A | 10 | 2 | - | - | 1 | - | - | 240 | 94,4 |
| 8B | 10 | 2 | - | - | 2 | - | - | 310 | 92,3 |
| 8C | 10 | 2 | - | - | 3 | - | - | 310 | 92,9 |
| 9A | 10 | 2 | - | - | - | 1 | - | 450 | 94,7 |
| 9B | 10 | 2 | - | - | - | 2 | - | 360 | 82,7 |
| 9C | 10 | 2 | - | - | - | 3 | - | 380 | 80,2 |
| 10A | 10 | 2 | - | - | - | - | 1 | 1300 | 83,4 |
| 10B | 10 | 2 | - | - | - | - | 2 | 1450 | 83,8 |
| 10C | 10 | 2 | - | - | - | - | 3 | 2150 | 84,8 |

### Herstellung der interpenetrierenden Netzwerke IPN

n-Butylacrylat wird mit 3 gew% (0,6 mol%) Poly(propylenglykol)dimethacrylat (Molgewicht 560 g/mol) in Gegenwart von 0,1 gew% AiBN wie oben beschrieben vemetzt. Der Film wird anschließend in THF gequollen, um unverbrauchtes Monomer herauszulösen, und dann wieder getrocknet. Dann lässt man den Film in einer Lösung des sternförmigen photoreaktiven Makromonomers in THF (10 gew%) aufquellen und anschließend wieder trocknen. Die Beladung des Netzwerks mit der photoreaktiven Komponente beträgt dann etwa 30 gew% .
Die polymeren amorphen Netzwerke wurden im Hinblick auf ihre weiteren thermischen und mechanischen Eigenschaften untersucht. Die Ergebnisse dieser Untersuchungen sind in der folgenden Tabelle zusammengefasst.

| Nr. | T_{g} (°C) | E-Modul E bei RT (MPa) | Reißspannung σᵣ bei RT (MPa) | Bruchdehnung εᵣ bei RT (%) |
|---|---|---|---|---|
| 1A | -40,8 | 0,54 | 0,24 | 45 |
| 1 B | -34,5 | 1,10 | 0,21 | 15 |
| 1C | -21,2 | 1,77 | 0,24 | 10 |
| 2A | -46,1 | 0,29 | 1,00 | 20 |
| 2B | -40,3 | 0,22 | 0,15 | 20 |
| 2C | -35,6 | 0,94 | 0,18 | 20 |
| 2D | -19,9 | 1,69 | 0,42 | 20 |
| 2E | -10,9 | 4,22 | 0,12 | 35 |
| 3A | -30,6 | 0,56 | 0,15 | 30 |
| 3B | -22,8 | 0,90 | 0,31 | 35 |
| 3C | -18,6 | 2,39 | 0,44 | 25 |
| 4A | -40,5 | 0,54 | 0,18 | 35 |
| 4B | -34,9 | 1,04 | 0,24 | 25 |
| 4C | -24,9 | 1,88 | 0,35 | 25 |
| 5A | -38,8 | 0,36 | 0,08 | 20 |
| 5B | -36,5 | 1,44 | 0,10 | 15 |
| 5C | -29,6 | 1,41 | 0,22 | 6 |
| 6A | -10, 0 | 1,80 | 0,34 | 25 |
| 6B | 2,2 | 11,52 | 2,48 | 35 |
| 6C | 16,1 | 120,69 | 9,66 | 15 |

| Nr. | T_{g} (°C) | E-Modul E bei RT (MPa) | Reißspannung σᵣ bei RT (MPa) | Bruchdehnung εᵣ bei RT (%) |
|---|---|---|---|---|
| 7A | -11,4 | 2,67 | 0,51 | 25 |
| 7B | 7,3 | 9,71 | 2,26 | 30 |
| 7C | 12,6 | 39,78 | 5,28 | 25 |
| 8A | -11, 9 | 2, 35 | 0,83 | 45 |
| 8B | 6,6 | 25,02 | 5,17 | 50 |
| 8C | 10,4 | 139,9 | 13,06 | 15 |
| 9A | 3,5 | 1,53 | 0, 53 | 50 |
| 9B | 8,5 | 14,04 | 4,55 | 60 |
| 9C | 13,9 | 32,42 | 6,42 | 50 |
| 10A | -27,4 25,7 | 1,40 | 0,29 | 30 |
| 10B | -23,6 52,8 | 2,41 | 0,67 | 25 |
| 10C | -20,0 56,6 | 4,74 | 0,96 | 25 |
| 11 * | -46,5 | 0,15 | > 1,60 | > 2000 |
| 12** vor Bestrahlung | -45,0 | 0,17 | 1.0-1,5 | 300-500 |
| 12 ** nach Bestrahl. | -40,0 | 0,20 | 0,5-0.9 | 30-100 |

| | | | | |
|---|---|---|---|---|
| * Netzwerk aus n-Butylacrylat; 0,3 mol% Vernetzer; ohne photoreaktive Komponente | | | | |
| ** IPN; 0,6 mol% Vernetzer, physikalisch beladen mit photoreaktiver Komponente | | | | |

Die Form-Gedächtnis-Eigenschaften wurden in zyklischen photomechanischen Experimenten bestimmt. Hierzu wurden ausgestanzte, hantelförmige 0,5 mm dicke Folienstücke mit einer Länge von 10 mm und einer Breite von 3 mm verwendet.

Ggf. wird das Material vor Beginn der photomechanischen Zyklen durch Bestrahlen mit λ₂ vorbehandelt, damit evtl. vorhandene Cylobutanringe gespalten werden und möglichst alle photoreaktiven Gruppen monomer vorliegen. Die Dehnung der Proben erfolgt mit einer Geschwindigkeit von 10 mm/min. Zur Fixierung der temporären Form wurden die Proben um 30 % gestreckt und bei konstanter Spannung bestrahlt. Zum Auslösen des Form-Gedächtnis-Effektes wurden die Proben spannungsfrei erneut bestrahlt.

Die Bestrahlung der Proben erfolgt mit einer UV-Lampe. Mit Hilfe eines Filters wird der richtige Wellenlängenbereich selektiert.
Normale Netzwerke mit CA : λ₁ = > 250 nm, λ₂ = < 250 nm
IPN mit CAA: λ₁ = > 300 nm; λ₂ = 254 nm
Der Abstand zur Probe betrug
10 cm, wenn eine 200 Watt Lampe verwendet wurde (> 300 nm), oder
3 cm, wenn eine 4 Watt Lampe zum Einsatz kam (254 nm), oder
10 cm, wenn eine 40 Watt Lampe verwendet wurde (> und < 250 nm).

Die optimale Bestrahlungsdauer hängt u.a. davon ab, wie groß der Abstand der Lampe zur Probe ist und wie groß die Lichtintensität ist. Für die normalen Netzwerke ist eine Bestrahlungsdauer von 30 min je Seite ausreichend, um maximal mögliche Werte für R_{f} und Rᵣ zu erzielen. Im Fall der IPNs wird ein maximaler Wert für R_{f} von 21 % nach 4 Stunden Bestrahlung erreicht.

Diese Experimente demonstrieren die überlegenen Eigenschaften der amorphen Netzwerke der vorliegenden Erfindung. Die Netzwerke zeichnen sich durch gute Werte für das die SMP-Eigenschaften kennzeichnende Gesamtrückstellverhältnis nach 5 Zyklen aus, wie die folgende Tabelle zeigt. Materialien des Stands der Technik zeigen hier häufig Werte von weniger als 80%.

Durch die einfachen Grundbausteine der erfindungsgemäßen Netzwerke ist darüber hinaus eine gewisse Einfachheit der Synthese sichergestellt. Durch Variieren der Zusammensetzung, wie oben demonstriert, können gezielt polymere Materialien erhalten werden, die sich durch erwünschte Eigenschaftskombinationen auszeichnen.

Die Materialien der vorliegenden Erfindung eignen sich insbesondere als Materialien auf dem medizinischen Gebiet, als Implantate, zur zielgesteuerten, stimuli-sensitiven Wirkstofffreisetzung, zur Bandaugmentation, als Bandscheibenersatz. Darüber hinaus sind die der amorphen Netzwerke oberhalb der Glasstemperatur transparent, was für bestimmte Anwendungen von Vorteil ist.

## Patentansprüche

1. Photosensitives polymeres Netzwerk, umfassend ein amorphes Netzwerk und eine photoreaktive Komponente.

2. Photosensitives polymeres Netzwerk nach Anspruch 1, wobei das amorphe Netzwerk eine Matrixkomponente und eine Vernetzerkomponente umfasst.

3. Photosensitives Netzwerk nach Anspruch 2, wobei die photoreaktive Komponente in das amorphe Netzwerk einpolymerisiert ist.

4. Photosensitives polymeres Netzwerk nach Anspruch 2, wobei die photoreaktive Komponente nicht in das amorphe Netzwerk einpolymerisiert ist.

5. Photosensitives polymeres Netzwerk nach Anspruch 4, wobei das polymere Netzwerk ein amorphes Netzwerk und eine physikalisch damit vermischte photoreaktive Komponente umfasst.

6. Photosensitives polymeres Netzwerk nach einem der vorstehenden Ansprüche, wobei die Matrixkomponente ein Acrylatmaterial und/oder ein Methacrylatmaterial ist und die Vernetzerkomponente eine Diacrylatverbindung und/oder eine Dimethacrylatverbindung ist.

7. Photosensitives polymeres Netzwerk nach einem der vorstehenden Ansprüche, wobei die photoreaktive Komponente eine zu einer reversiblen Photodimerisierungsreaktion fähige Komponente ist.

8. Photosensitives polymeres Netzwerk nach einem der vorstehenden Ansprüche, wobei die photoreaktive Komponente eine Zimtsäureesterverbindung oder eine Cinnamylsäureesterverbindung ist.

9. Photosensitives polymeres Netzwerk nach einem der vorstehenden Ansprüche, wobei die photoreaktive Komponente in der Form einer Acrylatverbindung in das amorphe Netzwerk einpolymerisiert wurde oder wobei die photoreaktive Komponente in der Form eines Polymeren oder Oligomeren mit mindestens drei photoreaktiven Gruppen mit dem amorphen Netzwerk physikalisch vermischt ist.

10. Verfahren zur Herstellung eines photosensitiven polymeren Netzwerks nach einem der vorstehenden Ansprüche, wobei entweder
- eine Matrixkomponente mit einer Vernetzerkomponente und einer photoreaktiven Komponente polymerisiert werden; oder
- eine Matrixkomponente mit einer Vernetzerkomponente zu einem amorphen Netzwerk polymerisiert werden, wonach eine photoreaktive Komponente physikalisch mit dem amorphen Netzwerk gemischt wird.

11. Verwendung eines photosensitiven polymeren Netzwerks nach einem der vorstehenden Ansprüche als medizinisches Material, insbesondere zum Transport und zur gezielten Freisetzung von Wirkstoffen oder Diagnostika.

12. Photoreaktive Komponente, umfassend ein oligomeres oder polymeres Grundgerüst mit mindestens drei Kettenenden, wobei jedes Kettenende eine photoreaktive Gruppe trägt.

13. Photoreaktive Komponente nach Anspruch 12, wobei die photoreaktive Gruppe eine zu einer reversiblen Photodimerisierungsreaktion fähige Gruppe ist.

14. Photoreaktive Komponente nach Anspruch 13, wobei die photoreaktive Gruppe eine Zimtsäureesterverbindung oder eine Cinnamylsäureesterverbindung ist.

15. Photoreaktive Komponente nach einem der Ansprüche 12 bis 14, wobei das Grundgerüst ein sternförmiges Grundgerüst mit drei bis sechs, bevorzugt vier Armen (Kettenenden) ist.

16. Photoreaktive Komponente nach Anspruch 15, wobei das Grundgerüst ein Polyalkylenglycolgrundgerüst, bevorzugt ein Polyethylenglycolgrundgerüst ist.

17. Verwendung einer photoreaktiven Komponente nach einem der Ansprüche 12 bis 16 zur Herstellung eines polymeren photosensitiven Netzwerks.

18. Verfahren zur Programmierung eines photosensitiven polymeren Netzwerks, umfassen die folgenden Stufen:
- Bereitstellen einer Probe eines photosensitiven polymeren Netzwerks, wobei die photoreaktiven Gruppen nicht in der photodimerisierten Form vorliegen,
- Verformung der Probe,
- Bestrahlung der Probe mit Licht einer Wellelänge, die die Photodimerisierung der photoreaktiven Komponente hervorruft,
- Entspannung der Probe.

19. Verfahren zur Programmierung eines photosensitiven polymeren Netzwerks nach Anspruch 18, wobei die photoreaktive Komponente eine Zimtsäureesterverbindung oder eine Cinnamylsäureesterverbindung ist.

20. Verfahren zur Programmierung eines photosensitiven polymeren Netzwerks nach Anspruch 18 oder 19, wobei das Licht UV-Strahlung ist mit einer Wellenlänge im Bereich von >250nm.

## Claims

1. A photosensitive polymeric network, comprising an amorphous network and a photoreactive component.

2. The photosensitive polymeric network in accordance with claim 1, wherein the amorphous network comprises a matrix component and a crosslinking component.

3. The photosensitive network in accordance with claim 2, wherein the photoreactive component is copolymerised with the amorphous network.

4. The photosensitive polymeric network in accordance with claim 2, wherein the photoreactive component is not copolymerised with the amorphous network.

5. The photosensitive polymeric network in accordance with claim 4, wherein the polymeric network comprises an amorphous network and a photoreactive component, physically admixed therewith.

6. The photosensitive polymeric network in accordance with any one of the preceding claims, wherein the matrix component is an acrylate material and/or a methacrylate material and wherein the crosslinking component is a diacrylate compound and/or a dimethacrylate compound.

7. The photosensitive polymeric network in accordance with any one of the preceding claims, wherein the photoreactive component is a component able to undergo a reversible photodimerization reaction.

8. The photosensitive polymeric network in accordance with any one of the preceding claims, wherein the photoreactive component is a cinnamic acid ester compound or a cinnamyl acid ester compound.

9. The photosensitive polymeric network in accordance with any one of the preceding claims, wherein the photoreactive compound is copolymerised with the amorphous network in the form of an acrylate compound or wherein the photoreactive component is physically admixed with the amorphous network in the form of a polymer or oligomer having at least three photoreactive groups.

10. A process for the preparation of a photosensitive polymeric network in accordance with any of the preceding claims, wherein either
- a matrix component is polymerised with a crosslinking component and a photoreactive component or
- a matrix component is polymerised with a crosslinking component to obtain an amorphous network, followed by physically admixing a photoreactive component with the amorphous network.

11. Use of a photosensitive polymeric network in accordance with any of the preceding claims as medicinal material, in particular for transportation and for targeted release of drugs or diagnostic agents.

12. A photoreactive component, comprising an oligomeric or polymeric scaffold with at least three chain terminals, wherein each chain terminal comprises a photoreactive group.

13. The photoreactive component according to claim 12, wherein the photoreactive group is a group able to undergo a reversible photodimerization reaction.

14. The photoreactive component in accordance with claim 13, wherein the photoreactive group is a cinnamic acid ester compound or a cinnamyl acid ester compound.

15. The photoreactive component in accordance with any of claims 12 to 14, wherein the scaffold is a star shaped scaffold with three to six, preferably four branches (chain terminals).

16. The photoreactive component in accordance with claim 15, wherein the scaffold is a polyalkylene glycol scaffold, preferably a polyethylene glycol scaffold.

17. Use of a photoreactive component in accordance with any of claims 12 to 16 for the preparation of a polymeric photosensitive network.

18. A process for programming a photosensitive polymeric network, comprising the following steps:
- providing a sample of a photosensitive polymeric network, wherein the photoreactive groups are not present in photodimerized form,
- deformation of the sample,
- irradiation of the sample with light having a wavelength initiating the photodimerization of the photoreactive component,
- relaxation of the sample.

19. The process for programming a photosensitive polymeric network in accordance with claim 18, wherein the photoreactive component is a cinnamic acid ester compound or a cinnamyl acid ester compound.

20. The process for programming a photosensitive polymeric network in accordance with claim 18 to 19, wherein the light is UV irradiation having a wavelength in the area of > 250 nm.

## Revendications

1. Réseau polymère photosensible comprenant un réseau amorphe et un composant photoréactif.

2. Réseau polymère photosensible selon la revendication 1, où le réseau amorphe .comprend un composant matriciel et un composant réticulant.

3. Réseau polymère photosensible selon la revendication 2, où le composant photoréactif est incorporé par polymérisation au réseau amorphe.

4. Réseau polymère photosensible selon la revendication 2, où le composant photoréactif n'est pas incorporé par polymérisation au réseau amorphe.

5. Réseau polymère photosensible selon la revendication 4, où le réseau polymère comprend un réseau amorphe et un composant photoréactif physiquement mélangé avec celui-ci.

6. Réseau polymère photosensible selon l'une des revendications précédentes, le composant matriciel est une matière acrylate et/ou une matière méthacrylate et le composant réticulant est un composé diacrylate et/ou un composé diméthacrylate.

7. Réseau polymère photosensible selon l'une des revendications précédentes, où le composant photoréactif est un composant capable d'une réaction de photodimérisation réversible.

8. Réseau polymère photosensible selon l'une des revendications précédentes, où le composant photoréactif est un composé ester de l'acide cinnamique ou un composé ester de l'acide cinnamylique.

9. Réseau polymère photosensible selon l'une des revendications précédentes, où le composant photoréactif sous forme d'un composé acrylate est incorporé par polymérisation au réseau amorphe, ou le composant photoréactif sous forme d'un polymère ou d'un oligomère présentant au moins trois groupes photoréactifs est mélangé physiquement avec le réseau amorphe.

10. Procédé pour la préparation d'un réseau polymère photosensible selon l'une des revendications précédentes, où soit
- on polymérise un composant matriciel avec un composant réticulant et un composant photoréactif ; soit
- on polymérise un composant matriciel avec un composant réticulant en vue de l'obtention d'un réseau amorphe, ensuite on mélange physiquement un composant photoréactif avec le réseau amorphe.

11. Utilisation d'un réseau polymère photosensible selon l'une des revendications précédentes en tant que matière médicale, en particulier pour le transport et pour la libération ciblée de principes actifs ou de réactifs diagnostiques.

12. Composant photoréactif, comprenant un squelette fondamental oligomère ou polymère avec au moins trois extrémités de chaîne, où chaque extrémité de chaîne porte un groupe photoréactif.

13. Composant photoréactif selon la revendication 12, le groupe photoréactif est un groupe capable d'une réaction de photodimérisation réversible.

14. Composant photoréactif selon la revendication 13, le groupe photoréactif est un composé ester de l'acide cinnamique ou un composé ester de l'acide cinnamylique.

15. Composant photoréactif selon l'une des revendications 12 à 14, où le squelette fondamental est un squelette fondamental en étoile avec 3 à 6 bras, de préférence avec 4 bras (extrémités de chaîne).

16. Composant photoréactif selon la revendication 15, où le squelette fondamental est un squelette fondamental polyalkylèneglycol, de préférence un squelette fondamental polyéthylène-glycol.

17. Utilisation d'un composant photoréactif selon l'une des revendications 12 à 16 pour la préparation d'un réseau polymère photosensible.

18. Procédé pour la programmation d'un réseau polymère photosensible comprenant les étapes suivantes :
- fourniture d'un échantillon d'un réseau polymère photosensible, les groupes photoréactifs ne se présentent pas sous forme photodimérisée,
- déformation de l'échantillon,
- irradiation de l'échantillon par une lumière d'une longueur d'onde, qui provoque la photodimérisation du composant photoréactif,
- détente de l'échantillon.

19. Procédé pour la programmation d'un réseau polymère photosensible selon la revendication 18, où le composant photoréactif est un composé ester de l'acide cinnamique ou un composé ester de l'acide cinnamylique.

20. Procédé pour la programmation d'un réseau polymère photosensible selon la revendication 18 ou 19, la où lumière est un rayonnement UV d'une longueur d'onde dans le domaine de >250 nm.
